(19) Europäisches Patentamt
European Patent Office
Office européen des brevets

(11) Publication number: **0 351 897**
**A2**

# (12) EUROPEAN PATENT APPLICATION

(21) Application number: **89201447.3**

(22) Date of filing: **06.06.89**

(30) Priority: **17.06.88 US 208197**

(43) Date of publication of application:
**24.01.90 Bulletin 90/04**

(84) Designated Contracting States:
**AT BE CH DE FR GB IT LI LU NL SE**

(51) Int. Cl.4: **A61K 47/00 , A61L 15/16**

(71) Applicant: **THE PROCTER & GAMBLE COMPANY**
**One Procter & Gamble Plaza**
**Cincinnati Ohio 45202(US)**

(72) Inventor: **Manring, Gary Lee**
**4269 Darr Drive**
**Oxford Ohio 45956(US)**
Inventor: **Smith, Ronald Lee**
**7588 Devonwood Drive**
**West Chester Ohio 45069(US)**

(74) Representative: **Suslic, Lydia et al**
**Procter & Gamble European Technical Center N.V. Temselaan 100**
**B-1820 Strombeek-Bever(BE)**

## (54) Skin penetration system for salts of amine-functional drugs.

(57) The invention involves pharmaceutical compositions for topical application comprising:

(a) a pharmaceutically-acceptable salt of addition of an amine-functional drug (other than opioid analgesic drugs);

(b) a $C_7$ to $C_{22}$ straight-chain or branched-chain, saturated or unsaturated, fatty acid having a melting point of less than about 50° C; and

(c) a $C_3$-$C_4$ alkane diol.

EP 0 351 897 A2

# SKIN PENETRATION SYSTEM FOR SALTS OF AMINE-FUNCTIONAL DRUGS

## TECHNICAL FIELD

The present invention relates to compositions for the topical administration of drugs, especially such compositions having a system for enhancing penetration of the drug through the skin.

## BACKGROUND OF THE INVENTION

For drugs to achieve therapeutic results, they must be administered in a manner such that the drug reaches the tissues of the body in need of treatment; in many instances such tissues are internal. Many routes for achieving internal delivery of drugs are available, and those preferred for various drugs are determined by many factors.

One route of internal delivery of drugs is by transdermal administration. Transdermal administration of drugs can be used in many instances to achieve therapeutic levels of the drugs in the systemic circulatory system, as well as for more localized internal dosing of drugs. Where such therapeutic levels of drugs can be achieved by transdermal administration, several potential advantages exist over other routes of administration. Sustained systemic delivery of drug controlled at therapeutic but below toxic levels over long periods of time with a single continuous application is often an advantage of transdermal drug administration. Potential contamination of internal tissues with undesired foreign substances or microbes, often associated with parenteral administration of drugs, is avoided with transdermal drug administration. Oral administration of many drugs is undesirable or unfeasible because the drug decomposes in the harsh environment of the gastrointestinal tract, lacks sufficient absorption from the gastrointestinal tract, or causes gastrointestinal upset or tissue damage in the gastrointestinal tract. First-pass metabolism of orally administered drugs can increase the dosage required to achieve therapeutic levels and thereby increase undesirable side effects either from the primary drug or the metabolites. Maintenance of uniform, optimal systemic levels of drugs for long periods of time is often difficult through oral administration. Such problems can often be reduced or avoided by transdermal drug administration.

Despite the substantial potential advantages for transdermal administration of drugs, relatively few drugs are so administered. The skin is a formidable barrier to the passage of most drugs. It is often necessary to provide a composition containing a skin penetration enhancing vehicle in order to provide sufficient transdermal penetration of the drug to achieve therapeutic levels of the drug at the target internal tissue. A number of skin penetration enhancing vehicles for drugs have been disclosed, including those in the following references: U.S. Patent No. 3,536,816 issued to Kellner on October 27, 1970; U.S. Patent No. 4,006,218 issued to Sipos on February 1, 1977; U.S. Patent No. 4,124,720 issued to Wenmaekers on November 7, 1978; U.S. Patent No. 4,126,681 issued to Reller on November 21, 1978; U.S. Patent No. 4,299,826 issued to Luedders on November 10, 1981; U.S. Patent No. 4,305,936 issued to Klein on December 15, 1981; U.S. Patent No. 4,309,414 issued to Inagi, Muramatsu & Nagai on January 5, 1982; U.S. Patent No. 4,338,306 issued to Kitao & Nishimura on July 6, 1982; U.S. Patent No. 4,442,090 issued to Kakeya, Kitao & Nishimura on April 10, 1984; U.S. Patent No. 4,485,033 issued to Kitao & Nishimura on November 27, 1984; U.S. Patent No. 4,537,776 issued to Cooper on August 27, 1985; U.S. Patent No. 4,552,872 issued to Cooper, Loomans & Fawzi on November 12, 1985; U.S. Patent No. 4,557,934 issued to Cooper on December 10, 1985; U.S. Patent No. 4,573,995 issued to Chen, Chun & Enscore on March 4, 1986; U.S. Patent No. 4,626,539 issued to Aungst & DiLuocio on December 2, 1986; U.S. Patent No. 4,637,930 issued to Konno, Kawata, Aruga, Sonobe & Mitomi issued January 20, 1987; U.S. Patent No. 4,695,465 issued to Kigasawa, Ohtani, Tanaka & Hayashida on September 22, 1987; European Patent Application No. 0,043,738 of The Procter & Gamble Company in the names of Wickett, Cooper & Loomans, published on June 13 1982; European Patent Application No. 0,095,813 of The Procter & Gamble Company in the name of Cooper, published December 7, 1983; PCT International Patent Application No. WO 87/03490 of Key Pharmaceuticals, Inc. in the names of Bodor and Loftson, published on June 18, 1987; Washitake, M., T. Anmo, I. Tanaka, T. Arita & M. Nakano, "Percutaneous Absorption of Drugs from Oily Vehicles", Journal of Pharmaceutical Sciences, Vol. 64, No. 3 (March, 1975), pp. 397-401; Shahi, V., & J. L. Zatz, "Effect of Formulation Factors on Penetration of Hydrocortisone through Mouse Skin", Journal of Pharmaceutical Sciences, Vol. 67, No. 6 (June, 1978), pp. 789-792; Cooper, E.R., "Increased Skin

Permeability for Lipophilic Molecules", Journal of Pharmaceutical Sciences, Vol. 73, No. 8 (August, 1984), pp. 1153-1156; Aungst, B.J., N. J. Rogers & E. Shefter, "Enhancement of Naloxone Penetration through Human Skin In Vitro Using Fatty Acids, Fatty Alcohols, Surfactants, Sulfoxides and Amides", International Journal of Pharmaceutics, Vol. 33 (1986), pp. 225-234; Green, P.G., & J. Hadgraft, "Facilitated Transfer of Cationic Drugs Across a Lipoidal Membrane by Oleic Acid and Lauric Acid", International Journal of Pharmaceutics, Vol. 37 (July, 1987), pp. 251-255.

U.S. Patent No. 4,552,872 discloses topical formulations with vehicles comprising a $C_3$-$C_4$ diol and a cell-envelope disordering compound which enhance the skin penetration of corticosteroids. European Patent Applications Nos. 0,043,738 and 0,095,813 disclose topical formulations with vehicles comprising combinations of diol compounds and cell envelope-disordering compounds which enhance the skin penetration of non-steroidal anti-inflammatory agents, local anesthetics, antibiotics, and antiviral compounds. U.S. Patent No. 4,626,539 discloses topical formulations with vehicles comprising propylene glycol combined with a fatty acid or a fatty alcohol which enhance the skin penetration of opioids.

Many drugs are amine-functional compounds. For many amine-functional drugs, the quantity of drug that can be transported through the skin in a given period of time is the primary factor which limits transdermal dosing of the drug. Sufficient skin penetration must occur in order to achieve a therapeutic dose of the drug in the target tissue. For most transdermal drug delivery systems, the base forms of the amine-functional drugs penetrate the skin more readily than salt forms of the drugs. However, the base forms of such amine-functional drugs are often less stable in transdermal compositions than the salt forms. Despite the number of transdermal delivery vehicles disclosed in the literature, there continues to be a need for more such vehicles in order to provide useful compositions for transdermal administration of more drugs.

It is an object of the present invention to provide novel compositions for enhancing the skin penetration of the salts of amine-functional drugs.

It is a further object of the present invention to provide such compositions which provide sufficient skin penetration enhancement to achieve therapeutic levels of the drugs in target internal tissues.

It is a still further object of the present invention to provide such compositions wherein the drugs have good stability.

## SUMMARY OF THE INVENTION

The present invention relates to pharmaceutical compositions for topical application comprising:

(a) a pharmaceutically-acceptable salt of addition of an amine-functional drug (other than opioid analgesic drugs);

(b) a $C_7$ to $C_{22}$ straight-chain or branched-chain, saturated or unsaturated, fatty acid having a melting point of less than about 50° C; and

(c) a $C_3$-$C_4$ alkane diol.

## DETAILED DESCRIPTION OF THE INVENTION

The present invention involves compositions comprising amine-functional drugs which may be applied topically to the skin and which result in transdermal penetration of the drugs through the skin. As used herein, an "amine-functional drug" is a compound which has a pharmaceutical effect on an internal tissue of the body and has the following chemical structure:

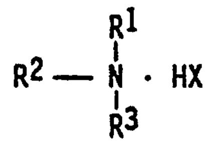

(1)

wherein each of $R^1$, $R^2$ and $R^3$ is independently selected from the group consisting of hydrogen (at most two of $R^1$, $R^2$, and $R^3$ can be hydrogen); unsubstituted or substituted, straight, branched or cyclic, aliphatic or heteroaliphatic chains; and two or all three of $R^1$, $R^2$ and $R^3$ being connected to form an unsubstituted or

substituted cyclic aliphatic or heteroaliphatic chain; and HX is an acid which associates with amine-functional compounds to form a pharmaceutically-acceptable salt of addition.

Many compounds having demonstrated pharmacological activity are amine-functional compounds. Many such pharmacologically active drugs which are amine-functional compounds, as defined hereinabove, are disclosed in the Merck Index, M. Windholz, (ed.), Merck & Company, Inc. (pub.), 10th Edition (1983) which is incorporated herein by reference. The present invention consists of pharmaceutical compositions comprising a pharmaceutically acceptable salt of such an amine-functional drug (other than opioid analgesic drugs). Following is a non-limiting, exemplary list of salts of amine-functional drugs which are included in the compositions of the present invention.

Compositions of the present invention include adrenergic drugs which are pharmaceutically-acceptable salts of amine-functional compounds. Adrenergic drugs preferred for inclusion in compositions of the present invention include pharmaceutically-acceptable salts of phenylephrine, phenylpropanolamine, ephedrine, pseudoephedrine, mephentermine, hydroxyamphetamine, isoproterenol, isometheptene, epinephrine, norepinephrine; dopamine, methoxyphenamine, protokylol, metaraminol, naphazoline, oxymetazoline, tetrahydrozoline, and xylometazoline. Adrenergic drugs more preferred for inclusion in compositions of the present invention include phenylephrine hydrochloride, phenylephrine tartrate, phenylephrine tannate, phenylpropanolamine hydrochloride, ephedrine hydrochloride, ephedrine sulfate, ephedrine tannate, pseudoephedrine hydrochloride, pseudoephedrine sulfate, mephentermine sulphate, hydroxyamphetamine hydrochloride, hydroxyamphetamine hydrobromide, isoproterenol hydrochloride, isoproterenol sulfate, isometheptene mucate, epinephrine tartrate, norepinephrine tartrate; dopamine hydrochloride, methoxyphenamine hydrochloride, protokylol hydrochloride, metaraminol tartrate, naphazoline hydrochloride, oxymetazoline hydrochloride, tetrahydrozoline hydrochloride, and xylometazoline hydrochloride. Adrenergic drugs more preferred still for inclusion in compositions of the present invention are phenylephrine hydrochloride, phenylephrine tartrate, phenylephrine tannate, phenylpropanolamine hydrochloride, ephedrine hydrochloride, ephedrine sulfate, ephedrine tannate, pseudoephedrine hydrochloride, pseudoephedrine sulfate, mephentermine sulphate, hydroxyamphetamine hydrochloride and hydroxyamphetamine hydrobromide. Adrenergic drugs most preferred for inclusion in compositions of the present invention are phenylephrine hydrochloride, phenylpropanolamine hydrochloride and pseudoephedrine hydrochloride.

Compositions of the present invention include brochodilator drugs which are pharmaceutically-acceptable salts of amine-functional compounds. Brochodilator drugs preferred for inclusion in compositions of the present invention include pharmaceutically-acceptable salts of albuterol, terbutaline, metaproterenol, isoetharine, ethylnorepinephrine, and bitolterol. Brochodilator drugs more preferred for inclusion in compositions of the present invention include albuterol sulfate, terbutaline sulfate, metaproterenol sulfate, metaproterenol hydrochloride, isoetharine hydrochloride, ethylnorepinephrine hydrochloride and bitolterol mesylate. Brochodilator drugs more preferred still for inclusion in compositions of the present invention include albuterol sulfate, terbutaline sulfate, metaproterenol sulfate, metaproterenol hydrochloride, and isoetharine hydrochloride. Brochodilator drugs most preferred for inclusion in compositions of the present invention are albuterol sulfate and terbutaline sulfate.

Compositions of the present invention include antihistaminic drugs which are pharmaceutically-acceptable salts of amine-functional compounds. Antihistaminic drugs preferred for inclusion in compositions of the present invention include pharmaceutically-acceptable salts of chlorpheniramine, triprolidine, diphenhydramine, doxylamine, pyrilamine, phenindamine, promethazine, cyproheptadine, azatadine, clemastine, carbinoxamine, tripelennamine, terfenadine, dexchlorpheniramine, brompheniramine, chlorcyclizine, diphenylpyraline, pheniramine and phenyltoloxamine. Antihistaminic drugs more preferred for inclusion in compositions of the present invention include chlorpheniramine maleate, chlorpheniramine tannate, triprolidine hydrochloride, triprolidine oxalate, diphenhydramine hydrochloride, diphenhydramine ascorbate, diphenhydramine citrate, doxylamine succinate, pyrilamine maleate, pyrilamine hydrochloride, pyrilamine tannate, phenindamine tartrate, promethazine hydrochloride, cyproheptadine hydrochloride, azatadine maleate, clemastine fumarate, carbinoxamine maleate, carbinoxamine hydrochloride, tripelennamine hydrochloride, tripelennamine citrate, pheniramine maleate, dexchlorpheniramine maleate, brompheniramine maleate, chlorcyclizine hydrochloride, diphenylpyraline hydrochloride, and phenyltoloxamine citrate. Antihistaminic drugs more preferred still for inclusion in compositions of the present invention include chlorpheniramine maleate, chlorpheniramine tannate, triprolidine hydrochloride, triprolidine oxalate, diphenhydramine hydrochloride, diphenhydramine ascorbate, diphenhydramine citrate, doxylamine succinate, pyrilamine maleate, pyrilamine hydrochloride, pyrilamine tannate, phenindamine tartrate, promethazine hydrochloride, cyproheptadine hydrochloride, azatadine maleate, clemas tine fumarate, carbinoxamine maleate, carbinoxamine hydrochloride, tripelennamine hydrochloride, tripelennamine citrate, dexchlorpheniramine maleate,

brompheniramine maleate and chlorcyclizine hydrochloride. Antihistaminic drugs most preferred for inclusion in compositions of the present invention include chlorpheniramine maleate, triprolidine hydrochloride and diphenhydramine hydrochloride.

Compositions of the present invention include antitussive drugs which are pharmaceutically-acceptable salts of amine-functional compounds. Antitussive drugs preferred for inclusion in compositions of the present invention include pharmaceutically-acceptable salts of dextromethorphan, codeine, caramiphen and carbetapentane. Antitussive drugs more preferred for inclusion in compositions of the present invention include dextromethorphan hydrobromide, codeine phosphate, codeine N-oxide hydrochloride, caramiphen edisylate, carbetapentane citrate and carbetapentane tannate. Antitussive drugs more preferred still for inclusion in compositions of the present invention include dextromethorphan hydrobromide, carbetapentane citrate, codeine phosphate and codeine N-oxide hydrochloride. Antitussive drugs most preferred for inclusion in compositions of the present invention include dextromethorphan hydrobromide.

Compositions of the present invention include antipruritic drugs which are pharmaceutically-acceptable salts of amine-functional compounds. Antipruritic drugs preferred for inclusion in compositions of the present invention include pharmaceutically-acceptable salts of methdilizine and trimeprazine. Antipruritic drugs more preferred for inclusion in compositions of the present invention include methdilizine hydrochloride and trimeprazine tartrate.

Compositions of the present invention include anticholinergic drugs which are pharmaceutically-acceptable salts of amine-functional compounds. Anticholinergic drugs preferred for inclusion in compositions of the present invention include pharmaceutically-acceptable salts of scopolamine, atropine, homatropine, levodopa, dicyclomine, hyoscyamine, procyclidine, trihexyphenidyl and ethopropazine. Anticholinergic drugs more preferred for inclusion in compositions of the present invention include scopolamine hydrobromide, scopolamine hydrochloride, atropine sulfate, atropine mucate, homatropine hydrobromide, homatropine hydrochloride, dicyclomine hydrochloride, hyoscyamine hydrobromide, hyoscyamine sulfate, procyclidine hydrochloride, trihexyphenidyl hydrochloride and ethopropazine hydrochloride. Anticholinergic drugs more preferred still for inclusion in compositions of the present invention include scopolamine hydrobromide, scopolamine hydrochloride, atropine sulfate, atropine mucate, homatropine hydrobromide and homatropine hydrochloride. Anticholinergic drugs most preferred for inclusion in compositions of the present invention include scopolamine hydrobromide and scopolamine hydrochloride.

Compositions of the present invention include anti-emetic and antinauseant drugs which are pharmaceutically acceptable salts of amine-functional compounds. Anti-emetic and antinauseant drugs preferred for inclusion in compositions of the present invention include pharmaceutically-acceptable salts of cyclizine, meclizine, chlorpromazine, buclizine, metoclopramide, prochlorperazine and trimethobenzamide. Anti-emetic and antinauseant drugs more preferred for inclusion in compositions of the present invention include cyclizine hydrochloride, meclizine hydrochloride, chlorpromazine hydrochloride, chlorpromazine maleate, buclizine hydrochloride, metoclopramide hydrochloride, prochlorperazine maleate and trimethobenzamide hydrochloride. Anti-emetic and antinauseant drugs more preferred still for inclusion in compositions of the present invention include cyclizine hydrochloride, meclizine hydrochloride, chlorpromazine hydrochloride and chlorpromazine maleate.

Compositions of the present invention include anorexic drugs which are pharmaceutically-acceptable salts of amine-functional compounds. Anorexic drugs preferred for inclusion in compositions of the present invention include pharmaceutically-acceptable salts of benzphetamine, phentermine, chlorphentermine, fenfluramine, diethylpropion and phendimetrazine. Anorexic drugs more preferred for inclusion in compositions of the present invention include benzphetamine hydrochloride, phentermine hydrochloride, chlorphentermine hydrochloride, fenfluramine hydrochloride, diethylpropion hydrochloride, phendimetrazine tartrate and phenmetrazine hydrochloride. Anorexic drugs more preferred still for inclusion in compositions of the present invention include benzphetamine hydrochloride, phentermine hydrochloride, chlorphentermine hydrochloride and fenfluramine hydrochloride.

Compositions of the present invention include central stimulant drugs which are pharmaceutically-acceptable salts of amine-functional compounds. Central stimulant drugs preferred for inclusion in compositions of the present invention include pharmaceutically-acceptable salts of amphetamine, methamphetamine, dextroamphetamine and methylphenidate. Central stimulant drugs more preferred for inclusion in compositions of the present invention include amphetamine sulfate, amphetamine succinate, amphetamine phosphate, methamphetamine hydrochloride, dextroamphetamine sulfate and methylphenidate hydrochloride. Central stimulant drugs more preferred still for inclusion in compositions of the present invention include amphetamine sulfate, amphetamine succinate, amphetamine phosphate, methamphetamine hydrochloride and dextroamphetamine sulfate.

Compositions of the present invention include antiarrhythmic drugs which are pharmaceutically-acceptable salts of amine-functional compounds. Antiarrhythmic drugs preferred for inclusion in compositions of the present invention include pharmaceutically-acceptable salts of propranolol, procainamide, disopyramide, quinidine, encainide, flecanaide, mexiletine and tocainide. Antiarrhythmic drugs more preferred for inclusion in compositions of the present invention include propranolol hydrochloride, procainamide hydrochloride, disopyramide phosphate, quinidine sulfate, quinidine gluconate, quinidine hydrochloride, quinidine hydrobromide, encainide hydrochloride, flecanide acetate, mexiletine hydrochloride and tocainide hydrochloride. Antiarrhythmic drugs more preferred still for inclusion in compositions of the present invention include propranolol hydro chloride, procainamide hydrochloride, quinidine sulfate and quinidine gluconate. Antiarrhythmic drugs most preferred still for inclusion in compositions of the present invention include propranolol hydrochloride.

Other antiarrhythmic drugs preferred for inclusion in compositions of the present invention include pharmaceutically-acceptable salts of the quinidine derivatives disclosed in U.S. Patent No. 4,716,171 issued to Jarreau and Koenig on December 29, 1987, which is hereby incorporated herein in its entirety by reference, having the following structure:

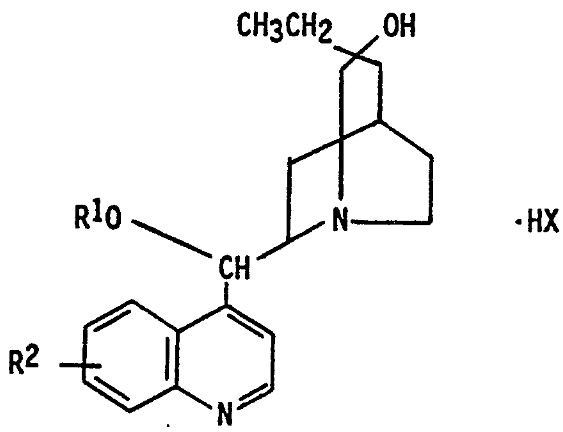

wherein $R^1$ represents a hydrogen atom, an alkyl group, or an acyl group; more preferably $R^1$ represents a hydrogen atom, an alkyl group with 1-4 carbon atoms, or an acyl group with 1-4 carbon atoms; most preferably $R^1$ is a hydrogen atom or an acetyl group; and $R^2$ represents a hydrogen atom, a hydroxy group, an alkoxy group, an acyloxy group or an aroyloxy group; more preferably $R^2$ represents a hydrogen atom, a hydroxy group, an alkoxy group with 1-4 carbon atoms, an acetoxy or formyloxy group, or a benzoyloxy group; most preferably $R^2$ is a hydrogen atom, hydroxy, methoxy, ethoxy, i-propoxy, n-propoxy or acetoxy; and HX is an acid which forms a salt of addition as defined hereinafter. $R^2$ can be at various positions of the quinolinyl nucleus; preferably $R^2$ occupies the 6′-position. Highly preferred compounds included in the compositions of the present invention include pharmaceutically-acceptable salts of 3S-hydroxy-10,11-dihydroquinidine, 3R-hydroxy-10,11-dihydroquinidine, 3R-hydroxy-O-acetyl-10,11-dihydroquini dine, and 3S-hydroxy-O-acetyl-10,11-dihydroquinidine, especially 3S-hydroxy-10,11-dihydroquinidine.

Compositions of the present invention include β-adrenergic blocker drugs which are pharmaceutically-acceptable salts of amine-functional compounds. β-Adrenergic blocker drugs preferred for inclusion in compositions of the present invention include pharmaceutically-acceptable salts of metoprolol, acebutolol, betaxolol, labetalol and timolol. β-Adrenergic blocker drugs more preferred for inclusion in compositions of the present invention include metoprolol tartrate, acebutolol hydrochloride, betaxolol hydrochloride, labetalol hydrochloride and timolol maleate. β-Adrenergic blocker drugs more preferred still for inclusion in compositions of the present invention include metoprolol tartrate.

Compositions of the present invention include cardiotonic drugs which are pharmaceutically-acceptable salts of amine-functional compounds. Cardiotonic drugs preferred for inclusion in compositions of the present invention include pharmaceutically-acceptable salts of milrinone, amrinone and dobutamine. Cardiotonic drugs more preferred for inclusion in compositions of the present invention include amrinone lactate and dobutamine hydrochloride.

Other cardiotonic drugs preferred for inclusion in compositions of the present invention include pharmaceutically-acceptable salts of 14-amino steroid derivatives, some of which are disclosed in U.S. Patent Nos. 4,325,879, 4,552,868 and 4,584,289, issued to Jarreau and Koenig on April 20, 1982, November 12, 1985 and April 22, 1986, respectively, each of which are hereby incorporated herein in their entirety by

reference. Preferred 14-amino steroid derivatives included in compositions of the present invention have the following structure:

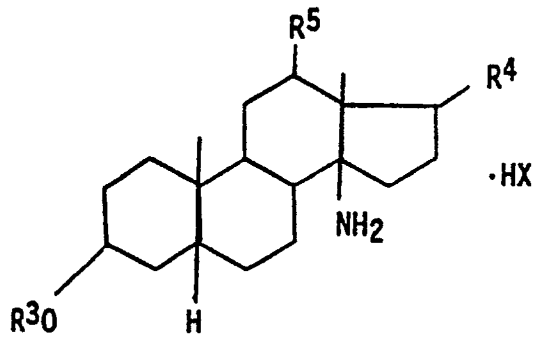

wherein $R^3$ represents a hydrogen atom, an alkyl group, an acyl group, or a substituted or unsubstituted sugar residue; $R^4$ represents a lower alkyl group substituted with hydroxy or lower alkoxy or carboxy and the lower alkyl esters thereof; $R^5$ represents a hydrogen atom, a hydroxyl group or an $-OR^6$ group, where $R^6$ is a substituted or unsubstituted sugar residue; and HX is an acid which forms a salt of addition as defined hereinafter.

$R^3$ is preferably a substituted or unsubstituted sugar residue: a substituted or unsubstituted monosaccharide residue, a substituted or unsubstituted oligosacchride residue or a substituted or unsubstituted polysaccharide. As nonlimiting examples, $R^3$ can be pentose or hexose modified or substituted in order to form 2-deoxy or 6-deoxy hexose, 2,6-dideoxy hexose, 2-deoxy 2-amino hexose, 3-deoxy 3-amino hexose, 3-deoxy 3-methoxy hexose, 2,3,6-trideoxy hexose, 4,6-dideoxy 4-methoxy hexose 2,3,6-trideoxy 2,3-didehydro hexose, etc. Examples of monosaccharides which may be used in the present invention include glucose, rhamnose, fructose, galactose, mannose, arabinose, digitoxose, cymarose, xylose, lyxose, ribose, digitalose, 6-deoxy glucose, glucosamine, 4-amino 2,4,6-trideoxy lyxohexopyranose, 4-amino 4,6-dideoxy glycopyranose, 2,3-dideoxy rhamnopyranose, 4-methoxy 4,6-dideoxy rhamnopyranose, etc., and preferably the $\beta$-D or $\alpha$L-anomers thereof. Disaccharides, such as saccharose, maltose or lactose or even various polysaccharides containing several sugars, may also be used in the present invention. Most preferred $R^3$ is a sugar residue selected from the group consisting of glucose, rhamnose, galactose, fructose and digitoxose.

Preferred $R^4$ is selected from the group consisting of $-CH(CH_3)OH$, $-CH(CH_3)OCH_3$ and $-CO_2CH_3$. Most preferred $R^4$ is $-CH(CH_3)OH$ or $-CO_2CH_3$. Preferred $R^5$ is hydrogen.

Compositions of the present invention include antihypertensive drugs which are pharmaceutically-acceptable salts of amine-functional compounds. Antihypertensive drugs preferred for inclusion in compositions of the present invention include pharmaceutically-acceptable salts of enalapril, clonidine, hydralazine, minoxidil (which is also a hair growth stimulator drug), guanadrel, guanethidine, guanfacine, mecamylamine, methyldopate, pargyline, phenoxybenzamine and prazosin. Antihypertensive drugs more preferred for inclusion in compositions of the present invention include enalapril maleate, clonidine hydrochloride, hydralazine hydrochloride, hydralazine sulfate, guanadrel sulfate, guanethidine sulfate, guanfacine hydrochloride, mecamylamine hydrochloride, methyldopate hydrochloride, pargyline hydrochloride, phenoxybenzamine hydrochloride and prazosin hydrochloride. Antihypertensive drugs more preferred still for inclusion in compositions of the present invention include enalapril maleate, clonidine hydrochloride, hydralazine hydrochloride and hydralazine sulfate. Antihypertensive drugs most preferred for inclusion in compositions of the present invention include clonidine hydrochloride.

Compositions of the present invention include diuretic drugs which are pharmaceutically-acceptable salts of amine-functional compounds. Diuretic drugs preferred for inclusion in compositions of the present invention include pharmaceutically-acceptable salts of amiloride and hydrochlorothiazide. Diuretic drugs more preferred for inclusion in compositions of the present invention include amiloride hydrochloride.

Compositions of the present invention include vasodilator drugs which are pharmaceutically-acceptable salts of amine-functional compounds. Vasodilator drugs preferred for inclusion in compositions of the present invention include pharmaceutically-acceptable salts of diltazem, amiodarone, isoxsuprine, nylidrin, tolazoline and verapamil. Vasodilator drugs more preferred for inclusion in compositions of the present invention include diltazem hydrochloride, amiodarone hydrochloride, isoxsuprine hydrochloride, nylidrin hydrochloride, tolazoline hydrochloride and verapamil hydrochloride. Vasodilator drugs more preferred still

for inclusion in compositions of the present invention include amiodarone hydrochloride.

Compositions of the present invention include vasoconstrictor drugs which are pharmaceutically-acceptable salts of amine-functional compounds. Vasoconstrictor drugs preferred for inclusion in compositions of the present invention include pharmaceutically-acceptable salts of dihydroergotamine, ergotamine and methysergide. Vasoconstrictor drugs more preferred for inclusion in compositions of the present invention include dihydroergotaimine mesylate, ergotamine tartrate and methysergide maleate.

Compositions of the present invention include anti-ulcer drugs which are pharmaceutically-acceptable salts of amine-functional compounds. Anti-ulcer drugs preferred for inclusion in compositions of the present invention include pharmaceutically-acceptable salts of ranitidine and cimetidine. Anti-ulcer drugs more preferred for inclusion in compositions of the present invention include ranitidine hydrochloride and cimetidine hydrochloride.

Compositions of the present invention include anesthetic drugs which are pharmaceutically-acceptable salts of amine-functional compounds. Anesthetic drugs preferred for inclusion in compositions of the present invention include pharmaceutically-acceptable salts of lidocaine, bupivacaine, chlorprocaine, dibucaine, etidocaine, mepivacaine, tetracaine, dyclonine, hexylcaine, procaine, cocaine, ketamine and pramoxine. Anesthetic drugs more preferred for inclusion in compositions of the present invention include lidocaine hydrochloride, bupivacaine hydrochloride, chlorprocaine hydrochloride, dibucaine hydrochloride, etidocaine hydrochloride, mepivacaine hydrochloride, tetracaine hydrochloride, dyclonine hydrochloride, hexylcaine hydrochloride, procaine hydrochloride, cocaine hydrochloride, ketamine hydro chloride and pramoxine hydrochloride. Anesthetic drugs more preferred still for inclusion in compositions of the present invention include lidocaine hydrochloride, bupivacaine hydrochloride, chlorprocaine hydrochloride, dibucaine hydrochloride, etidocaine hydrochloride, mepivacaine hydrochloride, tetracaine hydrochloride, dyclonine hydrochloride and hexylcaine hydrochloride.

Compositions of the present invention include antidepressant drugs which are pharmaceutically-acceptable salts of amine-functional compounds. Antidepressant drugs preferred for inclusion in compositions of the present invention include pharmaceutically-acceptable salts of imipramine, desipramine, amitriptyline, nortriptyline, protriptyline, doxepin, maprotiline, phenelzine, tranylcypromine, trazodone and trimipramine. Antidepressant drugs more preferred for inclusion in compositions of the present invention include imipramine hydrochloride, desipramine hydrochloride, amitriptyline hydrochloride, nortriptyline hydrochloride, protriptyline hydrochloride, doxepin hydrochloride, doxepin maleate, maprotiline hydrochloride, phenelzine sulfate, tranylcypromine sulfate, trazodone hydrochloride and trimipramine maleate.

Compositions of the present invention include tranquilizer and sedative drugs which are pharmaceutically-acceptable salts of amine-functional compounds. Tranquilizer and sedative drugs preferred for inclusion in compositions of the present invention include pharmaceutically-acceptable salts of chlordiazepoxide, benactyzine, benzquinamide, flurazepam, hydroxyzine, loxapine and promazine. Tranquilizer and sedative drugs more preferred for inclusion in compositions of the present invention include chlordiazepoxide hydrochloride, benactyzine hydrochloride, benzquinamide hydrochloride, flurazepam hydrochloride, hydroxyzine hydrochloride, loxapine hydrochloride, loxapine succinate and promazine hydrochloride. Tranquilizer and sedative drugs more preferred still for inclusion compositions of the present invention include chlordiazepoxide hydrochloride.

Compositions of the present invention include antipsychotic drugs which are pharmaceutically-acceptable salts of amine-functional compounds. Antipsychotic drugs preferred for inclusion in compositions of the present invention include pharmaceutically-acceptable salts of chlorprothixene, fluphenazine, haloperidol, molindone, thioridazine and trifluoperazine. Antipsychotic drugs more preferred for inclusion in compositions of the present invention include chlorprothixene hydrochloride, fluphenazine hydrochloride, fluphenazine decanoate, haloperidol hydrochloride, haloperidol decanoate, molindone hydrochloride, thioridazine hydrochloride and trifluoperazine hydrochloride.

Compositions of the present invention include antimicrobial drugs (antibacterial, antifungal, antiprotozoal and antiviral drugs) which are pharmaceutically-acceptable salts of amine-functional compounds. Antimicrobial drugs preferred for inclusion in compositions of the present invention include pharmaceutically-acceptable salts of $\beta$-lactam drugs, quinolone drugs, ciprofloxacin, norfloxacin, tetracycline, erythromycin, amikacin, doxycycline, capreomycin, chlorhexidine, chlortetracycline, oxytetracycline, clindamycin, ethambutol, metronidazole, pentamidine, gentamicin, kanamycin, lineomycin, methacycline, methenamine, minocycline, neomycin, netilmicin, paromomycin, streptomycin, tobramycin, miconazole and amanfadine. Antimicrobial drugs preferred for inclusion in compositions of the present invention include tetracycline hydrochloride, erythromycin estolate, erythromycin stearate (salt), amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole

hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride and amanfadine sulfate. Antimicrobial drugs more preferred still for inclusion in compositions of the present invention include tetracycline hydrochloride, erythromycin estolate, amantadine hydrochloride and amantadine sulfate.

Compositions of the present invention include antineoplastic drugs which are pharmaceutically-acceptable salts of amine-functional compounds. Antineoplastic drugs preferred for inclusion in compositions of the present invention include pharmaceutically-acceptable salts of bleomycin, daunorubicin, doxorubicin, mechlorethamine, procarbazine, quinacrine, tamoxifen, vinblastine and vincristine. Antineoplastic drugs more preferred for inclusion in compositions of the present invention include bleomycin sulfate, daunorubicin hydrochloride, doxorubicin hydrochloride, mechlorethamine hydrochloride, procarbazine hydrochloride, quinacrine hydrochloride, tamoxifen citrate, vinblastine sulfate and vincristine sulfate. Antineoplastic drugs more preferred still for inclusion in compositions of the present invention include daunorubicin hydrochloride, vinblastine sulfate and vincristine sulfate.

Compositions of the present invention include antimalarial drugs which are pharmaceutically-acceptable salts of amine-functional compounds. Antimalarial drugs preferred for inclusion in compositions of the present invention include pharmaceutically-acceptable salts of chloroquine, hydroxychloroquine primaquine and quinine. Antimalarial drugs more preferred for inclusion in compositions of the present invention include chloroquine phosphate, chloroquine sulfate, chloroquine hydrochloride, hydroxychloroquine sulphate, primaquine phosphate and quinine sulfate.

Compositions of the present invention include muscle relaxant drugs which are pharmaceutically-acceptable salts of amine-functional compounds. Muscle relaxant drugs preferred for inclusion in compositions of the present invention include pharmaceutically-acceptable salts of cinnamedrine, cyclobenzaprine, flavoxate, orphenadrine, papaverine, mebeverine, idaverine, ritodrine, dephenoxylate, dantrolene and azumolene. Muscle relaxant drugs more preferred for inclusion in compositions of the present invention include cinnamedrine hydrochloride, cyclobenz aprine hydrochloride, flavoxate hydrochloride, orphenadrine hydrochloride, orphenadrine citrate, papaverine hydrochloride, mebeverine hydrochloride, idaverine hydrochloride, ritodrine hydrochloride and dephenoxylate hydrochloride.

Compositions of the present invention include antispasmodic drugs which are pharmaceutically-acceptable salts of amine-functional compounds. Antispasmodic drugs preferred for inclusion in compositions of the present invention include pharmaceutically-acceptable salts of the compounds disclosed in U.S. Patent Number 3,856,825 issued to Wright, Burch and Goldenburg on December 24, 1974, which is hereby incorporated herein in its entirety by reference, which are 3-diethylamino-2,2-dimethylpropyl-5-(substituted phenyl)-2-furoates having the following chemical structure:

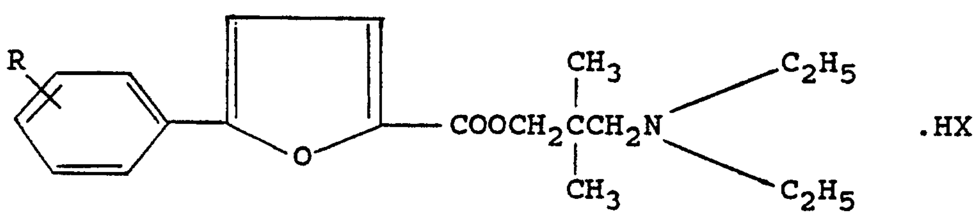

wherein R is a member of the group consisting of 4-nitro, 4-trifluoromethyl, 3,4-difluoro, 3-methoxy, 4-methyl, 4-methoxy, 4-bromo, 2,3-dichloro, 2-nitro-4-methyl, and 4-chloro; and HX is an acid salt of addition as defined hereinbefore. HX is preferably hydrochloride or fumarate. Most preferred is such compounds wherein R is 4-trifluoromethyl.

Compositions of the present invention include antidiarrheal drugs which are pharmaceutically-acceptable salts of amine-functional compounds. Antidiarrheal drugs preferred for inclusion in compositions of the present invention include pharmaceutically-acceptable salts of loperamide. Antidiarrheal drugs more preferred for inclusion in compositions of the present invention include loperamide hydrochloride.

Compositions of the present invention include bone-active drugs which are pharmaceutically-acceptable salts of amine-functional compounds. Bone-active drugs preferred for inclusion in compositions of the present invention include pharmaceu tically-acceptable salts of amine-functional diphosphonate drug compounds and phosphonoalkylphosphinate drug compounds, including the prodrug esters thereof. Such

compounds are disclosed, for example, in U.S. Patent Nos. 3,683,080 issued to Francis on August 8, 1972; 4,304,734 issued to Jary, Rihakova & Zobacova on December 8, 1981; 4,687,768 issued to Benedict & Johnson on August 18, 1987; 4,711,880 issued to Stahl & Schmitz on December 8, 1987; and 4,719,203 issued to Bosies & Gall on January 12, 1988; copending U.S. patent application Serial Nos. 808,584, of Benedict & Perkins filed December 13, 1985; 945,069 of Ebetino, Buckingham & McOsker filed December 19, 1986; 945,068 of Ebetino & Benedict filed December 19, 1986; and 069,666 of Ebetino filed July 6, 1987; and European Patent Application Nos. 0,001,584 of Blum, Hempel & Worms, published May 2, 1979; 0,039,033 published April 11, 1981; 0,186,405 of Benedict & Perkins, published July 2, 1986; and 0,243,173 of Oku, Todo, Kasahara, Nakamura, Kayakiri & Hashimoto, published October 28, 1987; all of which are hereby incorporated herein in their entirety by reference. Bone-active drugs more preferred in compositions of the present invention include pharmaceutically-acceptable salts of the following compounds or esters thereof: 6-amino-1-hydroxy-hexane-1,1-diphosphonic acid, 4-amino-1-hydroxy-butane-1,1-diphosphonic acid, N,N-dimethyl-4-amino-1-hydroxy-butane-1,1-diphosphonic acid, N,N-diethyl-4-amino-1-hydroxy-butane-1,1-diphosphonic acid, 3-amino-1-hydroxy-propane-1,1-diphosphonic acid, N,N-dimethyl-3-amino-1-hydroxy-propane-1,1-diphosphonic acid; N,N-diethyl-3-amino-1-hydroxy-propane-1,1-diphosphonic acid, 6-aminohexane-1,1-diphosphonic acid, phenylaminomethane diphosphonic acid, N,N-dimethylaminoethane diphosphonic acid, N-(2-hydroxyethyl)-aminomethane diphosphonic acid, N-acetylaminomethane diphosphonic acid, 3-(2-acetylaminocyclohexyl)-1-hydroxypropane-1,1-diphosphonic acid, 2- (2-aminomethylcyclohexyl)-1-hydroxyethane-1,1-diphosphonic acid, cis-3-(2-aminocyclohexyl)-1-hydroxypropane-1,1-diphosphonic acid, 3-(1-aminocyclohexyl)-1-hydroxypropane-1,1-diphosphonic acid, 3-(2-aminocyclohexyl)-1-hydroxypropane-1,1-diphosphonic acid, 3-(2-aminocyclopentyl)-1-hydroxypropane-1,1-diphosphonic acid, 1-aminoindan-2,2-diphosphonic acid, 4-(aminomethyl)-indan-2,2-diphosphonic acid, 1-(aminomethyl)-dihydro-2-pyrindine-6,6-diphosphonic acid, 4-aminohexahydroindan-2,2-diphosphonic acid, 4-(aminomethyl)-hexahydroindan-2,2-diphosphonic acid, octahydro-1-pyridine-5,5-diphosphonic acid; octahydro-2-pyridine-5,5-diphosphonic acid; octahydro-1-pyridine-6,6-diphosphonic acid; octahydro-2-pyridine-6,6-diphosphonic acid; octahydro-1-pyridine-7,7-diphosphonic acid; octahydro-2-pyridine-7,7-diphosphonic acid; 2-methyl-octahydro-1-pyridine-5,5-diphosphonic acid; 1,3-diethyl-octahydro-2-pyridine-5,5-diphosphonic acid; 7-hydroxy-octahydro-1-pyridine-6,6-diphosphonic acid; 4-methoxy-octahydro-2-pyridine-6,6-diphosphonic acid; 5-vinyl-octahydro-1-pyridine-7,7-diphosphonic acid; 1-(dimethylamino)-octahydro-2-pyridine-7,7-diphosphonic acid; N-acetyl-octahydro-2-pyridine-6,6-diphosphonic acid; N-benzyl-octahydro-1-pyridine-5,5-diphosphonic acid; N-(p-methoxyphenyl)-octahydro-2-pyridine-7,7-diphosphonic acid; 2-(3,4-dichlorophenyl)-octahydro-1-pyridine-7,7-diphosphonic acid; 2-(p-dimethylaminophenyl)-octahydro-1-pyridine-7,7-diphosphonic acid; 4-chloro-octahydro-1-pyridine-6,6-diphosphonic acid; 4-amino-octahydro-1-pyridine-6,6-diphosphonic acid; 7-carboxy-octahydro-1-pyridine-6,6-diphosphonic acid; 5-carboxy(methyl ester)-octahydro-1-pyridine-6,6-diphosphonic acid; 4-hydroxy-octahydro-2-pyridine-6,6-diphosphonic acid, propanoate ester; 4-(N,N-dimethylamino)-octahydro-1-pyridine-6,6-diphosphonic acid; 5-(N-acetamido)-octahydro-1-pyridine-7,7-diphosphonic acid; 7-(ethylketone)-octahydro-2-pyridine-5,5-diphosphonic acid; and 4-nitro-octahydro-1-pyridine-6,6-diphosphonic acid; (2′-piperidinyl)-methane diphosphonic acid; (3′-piperidinyl)-methane diphosphonic acid; (4′-piperidinyl)-methane diphosphonic acid; 2-(2′-piperidinyl)-ethane-1,1-diphosphonic acid; 2-(3′-piperidinyl)-ethane-1,1-diphosphonic acid; 2-(4′piperidinyl)ethane-1,1-diphosphonic acid; 2-(2′piperidinyl)-1-hydroxy-ethane-1,1-diphosphonic acid; 2-(3′-piperidinyl)-1-hydroxy-ethane-1,1-diphosphonic acid; 2-(4′-piperidinyl)-1-hydroxy-ethane-1,1-diphosphonic acid; 2-(2′-(3′methyl)-piperidinyl)-ethane-1,1-diphosphonic acid; 2-(2′-(5′-methyl)-piperidinyl)-ethane-1,1-diphosphonic acid; 2-(2′-(3′-methyl)- piperidinyl)-1-hydroxy-ethane-1,1-diphosphonic acid; 3-(2′-piperidinyl)-propane-1,1-diphosphonic acid; 3-(3′-piperidinyl)-propane-1,1-diphosphonic acid; 3-(4′-piperidinyl)-propane-1,1-diphosphonic acid; 3-(2′-piperidinyl)-1-hydroxy-propane-1,1-diphosphonic acid; 3-(3′-piperidinyl)-1-hydroxy-propane-1,1-diphosphonic acid; 3-(4′-piperidinyl)-1-hydroxy-propane-1,1-diphosphonic acid; 3-(2′-piperidinyl)-propane-2,2-diphosphonic acid; 3-(3′-piperidinyl)-propane-2,2-diphosphonic acid; 3-(4′-piperidinyl)-propane-2,2-diphosphonic acid; 2-(2′-(N-methyl)-piperidinyl)-1-hydroxy-ethane-1,1-diphosphonic acid; 2-(2′-piperidinyl)-1-amino-ethane-1,1-diphosphonic acid; 2-(3′-piperidinyl)-1-amino-ethane-1,1-diphosphonic acid; 2-(4′-piperidinyl)-1-amino-ethane-1,1-diphosphonic acid; 2-(2′-(3′-methyl)-piperidinyl)-1-amino-ethane-1,1-diphosphonic acid; 2-(2′-piperidinyl)-1-hydroxy-propane-1,1-diphosphonic acid; 3-(2′-piperidinyl)-propionic acid-2,2-diphosphonic acid; 2-(2′-piperidinyl)-1-(N-methyl)-amino-ethane-1,1-diphosphonic acid; 4-(2′-piperidinyl)-1-hydroxy-butane-1,1-diphosphonic acid; 2-(2′-(5′-amino)-piperidinyl)-1-hydroxy-ethane-1,1-diphosphonic acid; 2-(2′-(3′-ethyl)-piperidinyl)-1-hydroxy-ethane-1,1-diphosphonic acid; 2-(2′-(3′-carboxy)-piperidinyl)-1-hydroxy-ethane-1,1-diphosphonic acid; (2-(2′-(5′-carboxy)-piperidinyl)-1-hydroxy-ethane-1,1-diphosphonic acid; 2-(2′-(1′,4′-diazinyl))-ethane-1,1-diphosphonic acid; 2-(2′-(1′,4′-diazinyl))-1-hydroxy-ethane-1,1-diphosphonic acid; 2-(2′-(1′,3′-diazinyl))-ethane-1,1-diphosphonic acid; 2-(3′-(1′,2′-diazinyl))-ethane-1,1-diphosphonic acid; N-(2′-piperidinylidene)-amino-methane diphosphonic acid; N-(3′-piperidinyl)-amino-methane diphosphonic acid; N-(4′-piperidinyl)-amino-methane diphosphonic acid; N-(2′-(3′-methyl)-piperidinylidene)-amino-methane diphosphonic acid; N-(2′-(5′-methyl)-piperidinylidene)-amino-methane diphosphonic acid; 2-(N-(2′-piperidinylidene)-amino)-ethane-1,1-diphosphonic acid; 1-(N-(2′-piperidinylidene)-amino)-ethane-1,1-diphosphonic acid; N-(2′-(1′,3′-diazinylidene))-aminomethane diphosphonic acid; N-(2′(1′,4′-diazinylidene))-aminomethane diphosphonic acid; N-(2′-(1′,3′,5′-triazinylidene))-aminomethane diphosphonic acid; N-(4′-(1′,2′-diazinyl))- aminomethane diphosphonic acid; O-(3′piperidinyl)-oxamethane diphosphonic acid; O-(4′-piperidinyl)-oxamethane diphosphonic acid; 2-(O-(3′-piperidinyl)-oxa)-ethane-1,1-diphosphonic acid; 1-(O-(3′-piperidinyl)-oxa)-ethane-1,1-diphosphonic acid; O-(4′-(1′,2′-diazinyl))-oxamethane diphosphonic acid; S-(3′-piperidinyl)-thiomethane diphosphonic acid; S-(4′-piperidinyl)-thiomethane diphosphonic acid; 2-(S-(3′-piperidinyl)-thio)-ethane-1,1-diphosphonic acid; 1-(S-(3′-piperidinyl)-thio)-ethane-1,1-diphosphonic acid; S-(4′-(1′,2′-diazinyl))-thiomethane diphosphonic acid; N-(2-pyridyl)-aminomethane diphosphonic acid; N-(2-(5-amino)-pyridyl)-aminomethane diphosphonic acid; N-(2-(5-chloro)-pyridyl)-aminomethane diphosphonic acid; N-(2-(5-nitro)-pyridyl)-aminomethane diphosphonic acid; N-(2-(3,5-dichloro)-pyridyl)-aminomethane diphosphonic acid; N-(4-pyridyl)-N-ethyl-aminomethane diphosphonic acid; N-(2-(3-picolyl))-aminomethane diphosphonic acid; N-(2-(4-picolyl))-aminomethane diphosphonic acid; N-(2-(5-picolyl))-aminomethane diphosphonic acid; N-(2-(6-picolyl))-aminomethane diphosphonic acid; N-(2-(3,4-lutidine))-aminomethane diphosphonic acid; N-(2-(4,6-lutidine))-aminomethane diphosphonic acid; N-(2-pyrimidyl)-aminomethane diphosphonic acid; N-(4-(2,6-dimethyl)-pyrimidyl)-aminomethane diphosphonic acid; N-(2(4,6-dihydroxy)-pyrimidyl)-aminomethane diphosphonic acid; N-(2-(5-methoxy)-pyridyl)-aminomethane diphosphonic acid; N-(2-pyridyl)-2-aminoethane-1,1-diphosphonic acid; N-(2-(3-picolyl))-2-aminoethane-1,1-diphosphonic acid; N-(3-pyridyl)-2-amino-1-chloroethane-1,1-diphosphonic acid; N-(3-pyridyl)-2-amino-1-chloroethane-1,1-diphosphonic acid; N-(2-(4-picolyl))-2-amino-1-hydroxy-ethane-1,1-diphosphonic acid; (3-pyridyl)-aminomethane diphosphonic acid; 2-(2-pyridyl)-1-aminoethane-1,1-diphosphonic acid; O-(4-pyridyl)-1-amino-2-oxa-ethane-1,1-diphosphonic acid; N-(phenyl)-thiocarbamoylmethane diphosphonic acid; N-(4-chlorophenyl)carbamoylmethane diphosphonic acid; N-(4-chlorophenyl)thiocarbamoylmethane diphosphonic acid; N-(2-benzoyl-[b]thienyl)thiocarbamoylmethane diphosphonic acid); N-(4-trifluoromethylphenyl)thiocarbamoylmethane diphosphonic acid); N-(3-trifluoromethylphenyl)thiocarbamoylmethane diphosphonic acid); N-(4-chloro-3-trifluoromethylphenyl)-thiocarbamoylmethane diphosphonic acid); (4-chlorophenyl)sulfonylaminomethane diphosphonic acid); and N-(3-mesylaminophenyl)thiocarbamoylmethane diphosphonic acid). Bone-active drug compounds which are more preferred still in compositions of the present invention include pharmaceutically-acceptable salts of the following compounds or esters thereof: 6-amino-1-hydroxy-hexane-1,1-diphosphonic acid, 3-amino-1-hydroxy-propane-1,1-diphosphonic acid, octahydro-1-pyridine-6,6-diphosphonic acid, 2-(2′-piperidinyl)-ethane-1,1-diphosphonic acid; 2-(3′-piperidinyl)-ethane-1,1-diphosphonic acid; 2-(2′-piperidinyl)-1-hydroxy-ethane-1,1-diphosphonic acid; 2-(3′-piperidinyl)-1-hydroxy-ethane-1,1-diphosphonic acid; N-(2′-(3′-methyl)-piperidinylidene)-amino-methane diphosphonic acid; N-(2′-(1′,3′-diazinylidene))-aminomethane diphosphonic acid; and N-(2-(3-methylpiperidinylidene))-aminomethane-1,1-diphosphonic acid. Other preferred bone active drug compounds for use in the compositions of the present invention include the phosphonomethyl phosphinic acid compounds which are analogous to the above diphosphonic acid compounds.

As used herein, a "pharmaceutically-acceptable salt of an amine-functional compound" means a salt of addition of the compound having similar pharmacological activity (efficacy and safety) to the base form of the compound. Preferably a salt of addition of a compound has substantially the same pharmacological activity as the base form of the compound. A salt of addition of a compound can be formed by the association of an amine-functional drug compound in its base form with an appropriate acid. Acids known to form pharmaceutically-acceptable salts of addition with base forms of certain amine-functional drug compounds include, but are not limited to, the following: hydrochloric, hydrobromic, hydriodic, maleic, succinic, tartaric, fumaric, lactic, citric, ascorbic, oxalic, gluconic, phosphoric, nitric, sulfuric, methane sulfonic, ethane sulfonic and 2-naphthalene sulfonic.

The compositions of the present invention comprise a safe and effective amount of a salt of an amine-functional drug compound. The phrase "safe and effective amount", as used herein, means an amount of a drug high enough to significantly positively modify the condition to be treated, but low enough to avoid serious side effects (at a reasonable benefit/risk ratio), within the scope of sound medical judgement. A safe and effective amount of the drug will vary with the specific drug, the ability of the composition to penetrate the drug through the skin, the amount of composition to be applied, the particular condition being treated, the age and physical condition of the patient being treated, the severity of the condition, the duration of the treatment, the nature of concurrent therapy, and like factors.

The salts of amine-functional drug compounds present in the compositions of the present invention

preferably comprise from about 0.1% to about 60% by weight of the compositions, more preferably from about 1% to about 50%, more preferably still from about 5% to about 40%, and most preferably from about 10% to about 30%.

The vehicles of the compositions of the present invention significantly enhance the transdermal penetration of the salts of amine-functional drugs. The vehicles comprise, at a minimum, a fatty acid and a diol as described hereinafter.

Compositions of the present invention comprise a $C_7$-$C_{22}$ straight-chain or branched-chain, saturated or unsaturated, fatty acid having a melting point of less than about 50°C. Preferred fatty acids useful in the compositions of the present invention have a chain length of $C_{10}$-$C_{20}$, more preferably $C_{12}$-$C_{18}$. Preferred fatty acids are straight chain; preferred fatty acids are saturated, monounsaturated having a double bond, or diunsaturated having two double bonds. Particularly preferred fatty acids useful in the compositions of the present invention include lauric acid, oleic acid and linoleic acid; most preferred is oleic acid.

The fatty acids present in the compositions of the present invention preferably comprise from about 0.2% to about 10% by weight of the compositions, more preferably from about 0.5% to about 5%, and most preferably from about 1% to about 3%.

The compositions of the present invention also comprise a $C_3$-$C_4$ alkane diol. Preferred diol compounds useful in the com positions of the present invention include 1,2-propanediol, 1,3-propanediol, 1,2-butanediol, 1,3-butanediol, 1,4-butanediol, or mixtures of these diol compounds. In compositions of the present invention, 1,2-propanediol and 1,2-butanediol are more preferred diol compounds; 1,2-propanediol is an especially preferred diol compound.

The diols of the compositions of the present invention preferably comprise from about 20% to about 99% by weight of the compositions, more preferably from about 50% to about 95%, more preferably still from about 60% to about 95%, and most preferably from about 70% to about 90%.

Vehicles comprising diols and fatty compounds such as fatty acids, fatty alcohols and fatty acid esters have previously been known to be useful for enhancing transdermal penetration of drugs. See, for example, U.S. Patent Nos. 4,552,872 and 4,626,539 and European Patent Application Nos. 0,043,738 and 0,095,813. However, previous to Applicants invention, it was not known that the particular combination of a $C_3$-$C_4$ diol and the particular fatty acids disclosed hereinabove provides an unexpected and surprisingly great enhancement of the transdermal penetration of salts of amine-functional drugs.

Diols alone as a vehicle provide some transdermal penetration of drugs, and such penetration from a diol vehicle provides a convenient basis for comparing transdermal penetration of the same drug from vehicles consisting of the diol and a fatty component. For amine-functional drug compounds in their base form, a vehicle consisting of about 5% of a certain fatty acid (as described hereinabove) in a diol commonly provides, at best, a small increase in transdermal penetration of the drug compared to transdermal penetration of the drug from the diol alone (typically up to about a 5-fold increase in transdermal penetration). For salts of amine-functional drug compounds, such vehicle consisting of about 5% fatty acid in a diol provides a very substantial increase in transdermal penetration of the drug compared to its penetration from the diol alone (typically an increase in transdermal penetration of 10-fold to 100-fold or greater). Combining fatty alcohols or fatty acid esters with diols as vehicles for salts of amine-functional compounds does not provide the very substantial increase in transdermal penetration found to occur with the specific fatty acid/diol combination vehicles of the present invention.

## Test Method

The transdermal penetration of drugs is conveniently determined and compared from various vehicles using apparatus disclosed in Merritt, A.W. and E.R. Cooper, "In Vitro Diffusion Apparatus for Skin Penetration", Journal of Controlled Release, Volume 1 (1984), p. 151, which is hereby incorporated herein in its entirety, using the following procedure.

Dermatomed human back skin is obtained from cadavers after all hair had been clipped and the skin washed. The skin samples (approx. 0.25 mm in thickness) are then bathed in 10% glycerol and stored frozen. The glycerol prevents the formation of ice crystals which could possibly damage the keratinized cells and/or the intercellular lipid matrix. After a rapid thawing, the skin is washed with distilled water. Individual sections for use are selected based on uniform thickness (visual determination). An 11 mm diameter punch is used to section the selected areas.

Tests are conducted using glass diffusion cells placed in temperature-regulated stirring modules. Skin sections are mounted in the cells and receptor phase is added. The receptor phase is phosphate buffered

saline (pH 7.4) with 0.02% sodium azide added to inhibit microbial growth. It is preferable to allow the system to equilibrate overnight at 37 ± 2° C prior to dosing. Each diffusion cell has an exposed area of 0.7 $cm^2$ and a receptor capacity of 5 mL. Sufficient formulation is applied (approx. 150 ul) to ensure infinite dosing conditions. Receptor phase is replenished at each sampling time in order to maintain sink conditions. An additional 2 mL of receptor phase is used to rinse the cell compartment at each sampling (rinse added to the sample is collected). Preferably, five replicates are run with sampling intervals occurring at 4, 7, and 24 hours. Penetration rate is determined as the quantity of drug penetrating a measured area of skin per hour during the 7 to 24 hour interval, since steady state penetration is generally found to occur during that period. Penetration rate is usually expressed as µg drug per $cm^2$ skin per hour.

## EXAMPLES

The above test method is used to measure the transdermal penetration of salts of amine-functional drug compounds from compositions of the present invention. Penetration enhancement is the ratio of penetration rate of the drug from the fatty compound/diol combination to penetration rate of the drug from 100% diol. The results are reported in Table 1:

Table 1

| Example | Drug Compound | Diol | Fatty Acid | Fatty Acid: Diol Ratio | Penetration Enhancement |
|---|---|---|---|---|---|
| 1 | Phenyl-propanolamine hydrochloride | 1,2-Propane-diol | Oleic | 5:95 | 13.3 |
| 2 | Pseudoephedrine hydrochloride | 1,2-Propane-diol | Oleic | 5:95 | 78.1 |
| 3 | Phenylephrine hydrochloride | 1,2-Propane-diol | Oleic | 5:95 | 15.8 |
| 4 | Chlorpheniramine maleate | 1,2-Propane-diol | Oleic | 5:95 | 160<br>34.8 |
| 5 | Triprolidine hydrochloride | 1,2-Propane-diol | Oleic | 5:95 | 453 |
| 6 | Diphenhydramine hydrochloride | 1,2-Propane-diol | Oleic | 5:95 | 73.6 |
| 7 | Propranolol hydrochloride | 1,2-Propane-diol | Oleic | 5:95 | 353 |
| 8 | Clonidine hydrochloride | 1,2-Propane-diol | Oleic | 5:95 | 387 |
| 9 | Imipramine hydrochloride | 1,2-Propane-diol | Oleic | 5:95 | 318 |

Table 1 (continued)

| Example | Drug Compound | Diol | Fatty Acid | Fatty Acid: Diol Ratio | Penetration Enhancement |
|---|---|---|---|---|---|
| 10 | Phenylephrine hydrochloride | 1,2-Propane-diol | Lauric | 5:95 | 40.2 |

As a comparison, the above test method is also used to measure the transdermal penetration of the base form of amine-functional drug compounds from the same vehicles used in the compositions of the present invention, and to measure the transdermal penetration of salts of amine-function drug compounds from closely related vehicles. The results are reported in Table 2:

Table 2

| Example | Drug Compound | Diol | Fatty ComPound | Fatty Compound: Diol Ratio | Penetration Enhancement |
|---|---|---|---|---|---|
| 11 | Phenylpropanolamine base | 1,2-Propanediol | Oleic Acid | 5:95 | 1.3 |
| 12 | Pseudoephedrine base | 1,2-Propanediol | Oleic Acid | 5:95 | 3.4 |
| 13 | Chlorpheniramine base | 1,2-Propanediol | Oleic Acid | 5:95 | 0.9 |
| 14 | Doxylamine base | 1,2-Propanediol | Oleic Acid | 5:95 | 1.0 |
| 15 | Phenylephrinehydrochloride | 1,2-Propanediol | Oleyl Alcohol | 5:95 | 0.8 |
| 16 | Chlorpheniraminemaleate | 1,2-Propanediol | Methyl Oleate | 5:95 | 1.7 |

The compositions of the present invention may also comprise other pharmaceutically-acceptable carrier components. The term "pharmaceutically-acceptable carrier components", as used herein, means compatible solid or liquid filler diluents which are suitable for administration to a human or lower animal. The term "compatible", as used herein, means that the components are capable of being commingled with the drug compounds, diols and fatty acids of the compositions of the present invention, and with each other, in a manner such that there is no interaction which would substantially reduce the pharmaceutical efficacy of the compositions of the present invention under ordinary use situations. Pharmaceutically-acceptable carrier components must, of course, be of sufficiently high purity and sufficiently low toxicity to render them suitable for administration to the human or lower animal being treated.

Some examples of substances which can serve as pharmaceutically-acceptable carrier components are sugars, such as lactose, glucose and sucrose; starches such as corn starch and potato starch, cellulose and its derivatives, such as sodium carboxymethycellulose, ethylcellulose, hydroxypropylcellulose, nitrocellulose, cellulose acetate; powdered tragacanth; malt; gelatin; talc; paraffins; petrolatum; polyethylene glycol; glycerol; ethanol; water; antioxidants; surfactants; preservatives; thickeners; as well as other non-toxic compatible substances used in pharmaceutical formulations.

While particular embodiments of the present invention have been described, it will be obvious to those skilled in the art that various changes and modifications to the compositions disclosed herein can be made without departing from the spirit and the scope of the invention. It is intended to cover, in the appended claims, all such modifications that are within the scope of this invention.

## Claims

1. A pharmaceutical composition for topical application comprising a safe and effective amount, preferably from 0.1% to 60%, more preferably from 5% to 30% of a pharmaceutically-acceptable salt of addition of an amine-functional drug, other than an opioid analgesic drug, characterized in that it further comprises an amount, preferably from 0.2% to 10%, more preferably from 0.5% to 3% of a $C_7$ to $C_{22}$ straight-chain or branched-chain, saturated or unsaturated, fatty acid having a melting point of less than

50°C; and an amount, preferably from 20% to 99%, more preferably from 60% to 95% of a $C_3$-$C_4$ alkane diol, preferably 1,2-propanediol or 1,2-butanediol.

2. The composition of Claim 1 wherein said amine-functional drug is an adrenergic drug, preferably selected from phenylephrine hydrochloride, phenylephrine tartrate, phenylephrine tannate, phenylpropanolamine hydrochloride, ephedrine hydrochloride, ephedrine sulfate, ephedrine tannate, pseudoephedrine hydrochloride, pseudoephedrine sulfate, mephentermine sulphate, hydroxyamphetamine hydrochloride and hydroxyamphetamine hydrobromide.

3. The composition of Claim 1 wherein said amine-functional drug is a bronchodilator drug, preferably selected from albuterol sulfate, terbutaline sulfate, metaproterenol sulfate, metaproterenol hydrochloride and isoetharine hydrochloride.

4. The composition of Claim 1 wherein said amine-functional drug is an antihistaminic drug, preferably selected from chlorpheniramine maleate, chlorpheniramine tannate, triprolidine hydrochloride, triprolidine oxalate, diphenhydramine hydrochloride, diphenhydramine ascorbate, diphenhydramine citrate, doxylamine succinate, pyrilamine maleate, pyrilamine hydrochloride, pyrilamine tannate, phenindamine tartrate, promethazine hydrochloride, cyproheptadine hydrochloride, azatadine maleate, clemastine fumarate, carbinoxamine maleate, carbinoxamine hydrochloride, tripelennamine hydrochloride, tripelennamine citrate, dexchlorpheniramine maleate, brompheniramine maleate and chlorcyclizine hydrochloride.

5. The composition of Claim 1 wherein said amine-functional drug is an antitussive drug, preferably selected from dextromethorphan hydrobromide, carbetapentane citrate, codeine phosphate and codeine N-oxide hydrochloride.

6. The composition of Claim 1 wherein said amine-functional drug is an anticholinergic drug, preferably selected from scopolamine hydrobromide, scopolamine hydrochloride, atropine sulfate, atropine mucate, homatropine hydrobromide and homatropine hydrochloride.

7. The composition of Claim 1 wherein said amine-functional drug is an anti-emetic or antinauseant drug, preferably selected from cyclizine hydrochloride, meclizine hydrochloride, chlorpromazine hydrochloride and chlorpromazine maleate.

8. The composition of Claim 1 wherein said amine-functional drug is an antiarrhythmic or antihypertensive drug, preferably selected from propranolol hydrochloride, procainamide hydrochloride, quinidine sulfate, quinidine gluconate, enalapril maleate, clonidine hydrochloride, hydralazine hydrochloride, hydralazine sulfate, and salts of addition of 3S-hydroxy-10,11-dihydroquinidine, 3R-hydroxy-10,11-dihydroquinidine, 3R-hydroxy-O-acetyl-10,11-dihydroquinidine, and 3S-hydroxy-O-acetyl-10,11-dihydroquinidine.

9. The composition of Claim 1 wherein said amine-functional drug is an anesthetic or antipruritic drug, preferably selected from lidocaine hydrochloride, bupivacaine hydrochloride, chlorprocaine hydrochloride, dibucaine hydrochloride, etidocaine hydrochloride, mepivacaine hydrochloride, tetracaine hydrochloride, dyclonine hydrochloride and hexylcaine hydrochloride.

10. The composition of any of Claims 1-9 wherein said fatty acid is a $C_{10}$-$C_{20}$ straight-chain compound, preferably a $C_{12}$-$C_{18}$ straight-chain compound, saturated or mono- or di-unsaturated having 1 or 2 double bonds, especially oleic acid.